# EUROPEAN PATENT SPECIFICATION

(11) **EP 3 369 391 B1**
(45) Date of publication and mention of the grant of the patent: **15.07.2020**
(21) Application number: 16859688.0
(22) Date of filing: 20.10.2016
(51) Int. Cl.: A61B 17/56, A61G 13/00

(54) **DEVICE FOR PATIENT BODY POSITIONING AND IMMOBILIZING, AND ANGLE INDICATOR**
VORRICHTUNG ZUR POSITIONIERUNG UND FIXIERUNG EINES PATIENTENKÖRPERS SOWIE WINKELGEBER
DISPOSITIF DE MAINTIEN DE POSTURE ET INDICATEUR D'ANGLE

(30) Priority: 29.10.2015 JP 2015213110
(43) Date of publication of application: 05.09.2018
(73) Proprietor: LEXI Co., Ltd., Tokyo 170-0002 (JP)
(72) Inventor: IWAKIRI, Kentaro, Ikoma-shi Nara 630-0136 (JP)
(74) Representative: TBK
(86) International application number: PCT/JP2016/081151
(87) International publication number: WO 2017/073458

(56) References cited:
- CN-A- 102 309 392
- JP-A- 2000 060 883
- JP-A- 2011 502 626
- JP-A- 2014 069 080
- JP-B1- 5 956 047
- US-B1- 6 311 349

## Description

### Technical Field

The present invention relates to a body position fixing apparatus that fixes a human body in a lateral position and an angle indicator that supports a treatment on a human body in a lateral position.

### Background Art

A hip joint constituting a part of human lower extremities is a particularly important joint for walking, etc. in daily life. In addition, the hip joint is a region which is apt to suffer traumatic injury, has a joint cartilage that is subjected to degenerative destruction with aging, and often leads to arthroplasty.

As a surgical treatment for the hip joint, total hip arthroplasty (THA) is widely practiced. In THA, a cup is attached to an acetabulum of the pelvis and a stem is attached to a femur. Then, a liner formed of super-high molecular polyethylene or the like is received in the cup, and a femur head sphere attached to the tip of the stem is received by the liner.

JP 2000 060883 A discloses a body position fixing apparatus having the features of the preamble of claim 1. JP 2011 502626 A discloses an angle indicator that supports a treatment on a human in a lateral position.

### Summary of the Invention

### [Technical Problem]

In the treatment of THA, the patient's posture is generally in the lateral position. The treatment in the lateral position has advantages in that the operative field is easy to see and the legs can be moved in a wide range during the treatment. On the other hand, the treatment in the lateral position has disadvantages in that the anteroposterior inclination of the pelvis tends to vary, and since pelvic movement also occurs during surgery, the target reference surface in the cup placement becomes unclear, and the cup placement accuracy is insufficient.

It is an object of the present invention to provide a body position fixing apparatus and an angle indicator capable of improving an accuracy of a treatment such as THA.

### [Solution to Problem]

The above object is achieved by a body position fixing apparatus having the features of claim 1 or an angle indicator having the features of claim 13. Advantageous further developments are set out in the dependent claims.

### [Advantages of the Invention]

According to the present invention, the accuracy of a treatment such as THA can be improved. For example, in the THA performed in the lateral position, the cup placement accuracy can be improved. Other effects obtained from the present invention will be apparent from the following disclosure.

### Brief Description of Drawings

FIG. 1 is a perspective view showing a configuration example of a body position fixing apparatus according to an embodiment.
FIG. 2 is a diagram showing a lumbar skeleton of a human body.
FIG. 3 is a diagram showing an example of a hip prosthesis.
FIG. 4 is a schematic front view of a fixing device included in the body position fixing apparatus.
FIG. 5 is a schematic side view of the fixing device.
FIG. 6 is a view schematically showing pressing surfaces and a fixed reference plane of the fixing device.
FIG. 7 is a schematic front view of an angle indicator included in the body position fixing apparatus.
FIG. 8 is a schematic plan view of the angle indicator.
FIG. 9 is a diagram schematically showing an aspect of use of the body position fixing apparatus.
FIG. 10 is a graph showing measurement results of anteversion angles and inclination angles of a cup placed without using the angle indicator.
FIG. 11 is a graph showing measurement results of anteversion angles and inclination angles of a cup placed using the angle indicator.

### Description of Embodiments

Hereinafter, an embodiment will be described with reference to the drawings.

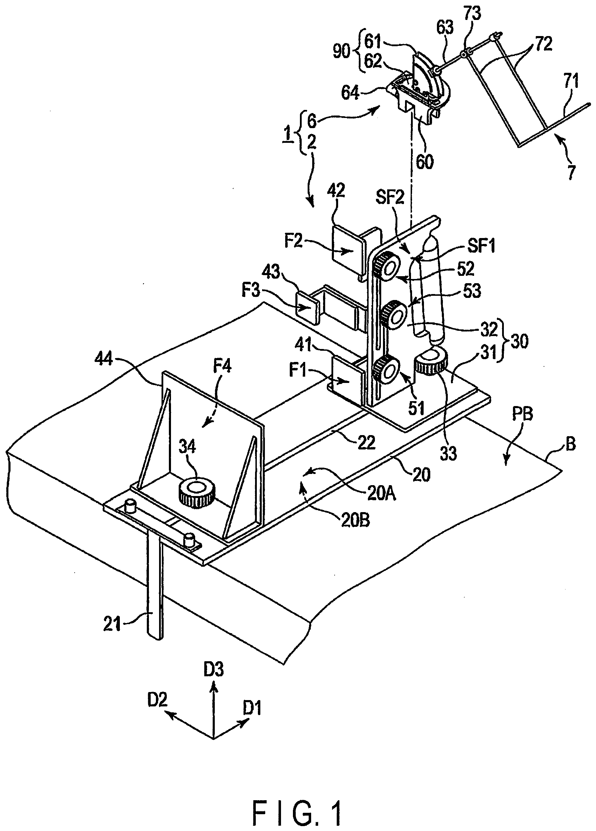

FIG. 1 is a perspective view showing a configuration example of a body position fixing apparatus according to this embodiment.

The illustrated body position fixing apparatus 1 includes a fixing device 2 and an angle indicator 6. The fixing device 2 is mounted on a bed B on which a patient is placed. The angle indicator 6 is attachable to and detachable from the fixing device 2, and is shown in a detached state in FIG. 1.

The fixing device 2 includes a fixing base 20 (a second base). In the example of FIG. 1, the fixing base 20 is an elongated metal plate including an upper surface 20A (first surface) and a bottom surface 20B parallel to each other; for example, placed on a placement surface PB of the bed B. In the following description, a length direction of the fixing base 20 is defined as a first direction D1, a width direction of the fixing base 20 is defined as a second direction D2, and a direction intersecting the directions D1 and D2 is defined as a third direction D3. In the present embodiment, the directions D1 to D3 intersect at right angles. However, the directions D1 to D3 may be directions intersecting at angles other than right angles.

In the example of FIG. 1, the fixing base 20 is installed so that the bottom surface 20B comes into surface contact with the bed B and its length direction coincides with the width direction of the bed B. In other words, in a state in which the fixing device 2 is attached to the bed B, the first direction D1 coincides with the width direction of the bed B, the second direction D2 coincides with the length direction of the bed B, and the third direction D3 coincides with the normal direction of the placement surface PB. At this time, both the upper surface 20A and the bottom surface 20B are parallel to the placement surface PB.

A guide 21 extending downward along the third direction D3 is attached to one end portion of the fixing base 20. The guide 21 supports attachment of the fixing device 2 to the bed B. The fixing base 20 has a guide groove 22 parallel to the first direction D1 in the upper surface 20A.

The fixing device 2 further includes a stand 30, a first pressing tool 41, a second pressing tool 42, and a third pressing tool 43. In the example of FIG. 1, the stand 30 has a first portion 31 in surface contact with the upper surface 20A of the fixing base 20 and a second portion 32 connected to the first portion 31 and extending in the third direction D3. Each of the first portion 31 and the second portion 32 is, for example, a metal plate and they are joined in a T shape.

Specifically, the second portion 32 is a plate including a first side face SF1 and a second side face SF2 on the side opposite to the first side face SF1. Both the first side face SF1 and the second side face SF2 are planes parallel to the first direction D1 and the third direction D3.

The stand 30 is fixed to the fixed base 20 by a fixing member 33. The fixing member 33 is, for example, a knob including a male screw threaded through a through hole provided in the first portion 31. The male screw of the knob is tightened to, for example, a female screw provided in the guide groove 22. By rotating the knob, the fixation between the stand 30 and the fixing base 20 is released, and the stand 30 can be slid along the guide groove 22.

The first pressing tool 41, the second pressing tool 42, and the third pressing tool 43 are attached to the stand 30 by a first support mechanism 51, a second support mechanism 52, and a third support mechanism 53, respectively. The first pressing tool 41, the second pressing tool 42, and the third pressing tool 43 respectively have a first pressing surface F1, a second pressing surface F2, and a third pressing surface F3. The pressing surfaces F1 to F3 are, for example, surfaces parallel to the second direction D2 and the third direction D3.

The fixing device 2 further includes a fourth pressing tool 44. In the example of FIG. 1, the fourth pressing tool 44 is a metal plate bent in an L shape and fixed to the fixing base 20 by a fixing member 34. Like the fixing member 33 described above, the fixing member 34 is, for example, a knob including a male screw threaded through a through hole provided in the fourth pressing tool 44. The male screw of the knob is tightened to, for example, a female screw provided in the guide groove 22. By rotating the knob, the fixation between the fourth pressing tool 44 and the fixing base 20 is released, and the fourth pressing tool 44 can be slid along the guide groove 22.

The fourth pressing tool 44 has a fourth pressing surface F4. The fourth pressing surface F4 is a surface parallel to the second direction D2 and the third direction D3, for example, and faces the pressing surfaces F1 to F3.

The angle indicator 6 includes an indicator base 60 (first base), a first angle adjuster 61, a second angle adjuster 62, an indicating rod 63, and a base fixing mechanism 64. One end of the indicating rod 63 is pivotally supported by the first angle adjuster 61. The first angle adjuster 61 is pivotally supported by the second angle adjuster 62. The second angle adjuster 62 is attached to the indicator base 60. The indicator base 60 is attached to the second portion 32 of the stand 30 by the base fixing mechanism 64.

The angle adjusters 61 and 62 constitute a rotation mechanism 90 that supports the indicating rod 63 to be rotatable at a discretionary angle with respect to a plane including the pressing surfaces F1 to F4 (an anatomical pelvic plane P1 or a fixed reference plane P2 described later) and the placement surface PB or the like.

In the example of FIG. 1, the angle indicator 6 further includes an assisting tool 7 pivotally supported by the indicating rod 63. The assisting tool 7 includes an assisting rod 71, two shafts 72 that attach the assisting rod 71 to the indicating rod 63, and a rod fixing mechanism 73.

One end of each shaft 72 is connected to the assisting rod 71, the other end is provided with an opening, and the indicating rod 63 is passed through the openings of the shafts 72. The shafts 72 have the same length and are perpendicular to the indicating rod 63 and the assisting rod 71. Therefore, the assisting rod 71 is parallel to the indicating rod 63.

The rod fixing mechanism 73 is provided on one shaft 72, and fixes the assisting tool 7 to the indicating rod 63. The rod fixing mechanism 73 is, for example, a knob including a male screw inserted into a female screw provided in the shaft 72. By fastening the knob, the tip of the male screw is pressed against the indicating rod 63, and the assisting tool 7 can be fixed to the indicating rod 63. By loosening the knob, fixation between the assisting tool 7 and the indicating rod 63 can be released. In a state in which the fixation is released, the assisting tool 7 can be moved along the indicating rod 63, or the assisting rod 71 can be rotated about the indicating rod 63 as an axis.

The body position fixing apparatus 1 having the above-described configuration is used mainly to fix the lumbar region of a patient who is placed on the placement surface PB of the bed B in the lateral position when performing THA, for example.

Here, an outline of the THA and fixation of the human body by the fixing device 2 will be described.

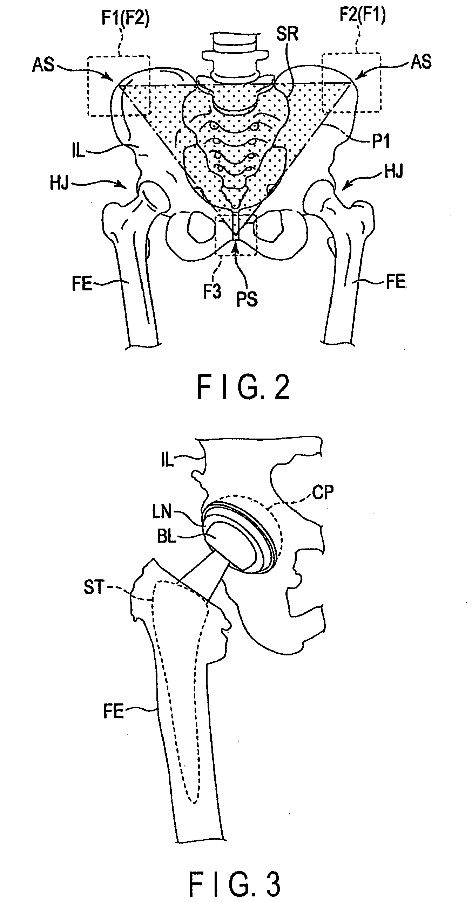

FIG. 2 shows a lumbar skeleton of the human body. In THA, a hip joint HJ connecting an ilium IL and a femur FE is replaced with a hip prosthesis.

FIG. 3 shows an example of a hip prosthesis. The illustrated hip prosthesis includes a cup CP placed in the acetabulum of the ilium IL and a stem ST attached to the femur FE. A liner LN is fitted in the cup CP, and a femur head sphere BL attached to the tip of the stem ST is fitted in the liner LN.

As shown in FIG. 2, the first pressing surface F1 is pushed from the front of the lumbar region of one of paired anterior superior iliac spines AS of the ilium IL, which is closer to the placement surface PB. The second pressing surface F2 is pushed from the front of the lumbar region of one of the paired anterior superior iliac spines AS, which is more distant from the placement surface PB. The third pressing surface F3 is pressed against the pubic symphysis PS from the front of the lumbar region. The fourth pressing surface F4 is pressed against the sacrum SR from the back of the lumbar region.

Since each of the pressing surfaces F1 to F4 is pressed against the human body from the outside, fixation itself by the fixing device 2 is non-invasive. By adjusting the positions of the stand 30 and the fourth pressing tool 44 along the guide groove 22, it is possible to press the pressing surfaces F1 to F4 against the respective parts with suitable pressure regardless of the body thickness of the lumbar region.

In order for the hip prosthesis to function properly, the placement angle of the cup CP is important. That is, the normal direction (symmetry axis) of the hemispherical cup CP must coincide with the target angle set for each patient in preoperative planning. The target angle can be set with reference to an anatomical pelvic plane (P1) including a pair of anterior superior iliac spines AS and a pubic symphysis PS. However, the target angle may be set with reference to other planes.

Subsequently, details of the fixing device 2 will be described.

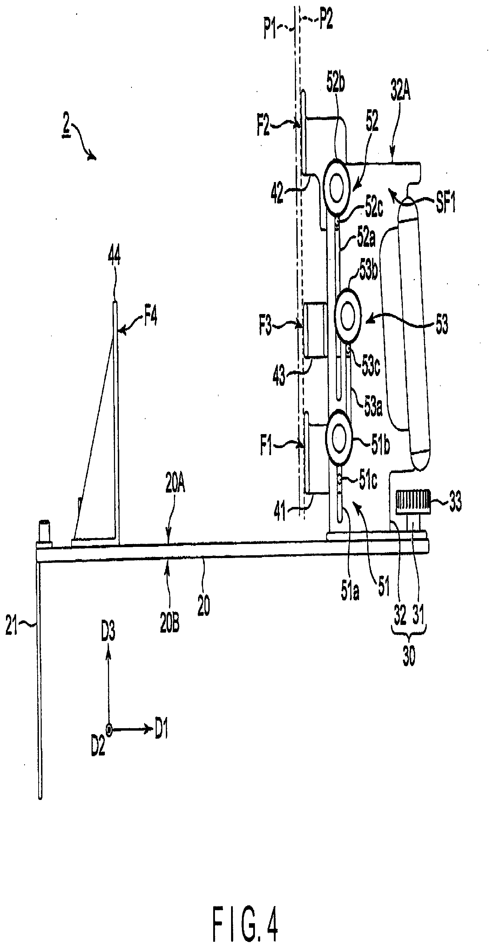

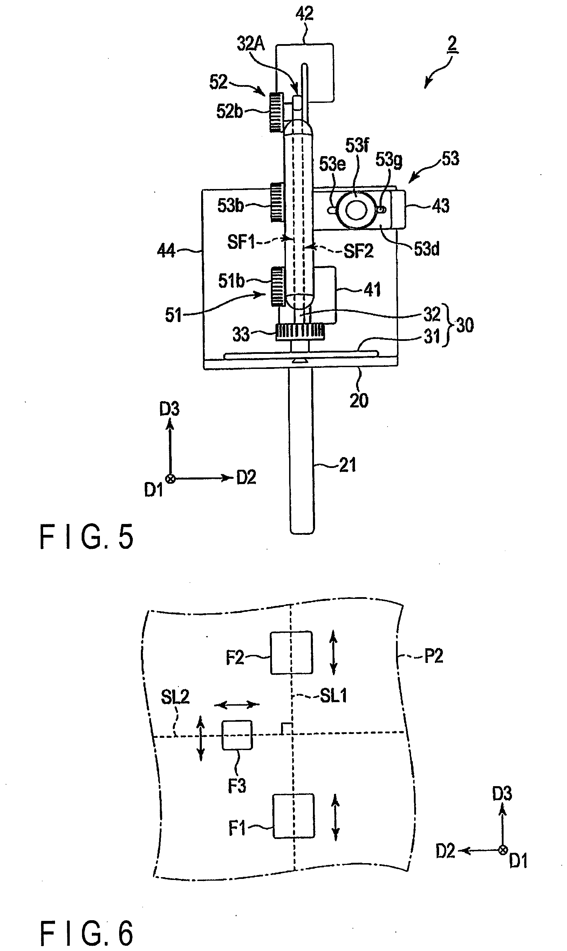

FIG. 4 is a schematic front view of the fixing device 2, and FIG. 5 is a schematic side view of the fixing device 2 as viewed from the stand 30 side. As shown in FIG. 4, the first pressing surface F1, the second pressing surface F2, and the third pressing surface F3 are located in the same plane. Hereinafter, a plane including the pressing surfaces F1 to F3 is referred to as the fixed reference plane P2. When the pressing surfaces F1 to F3 are accurately pressed against the pair of anterior superior iliac spines and the pubic symphysis, respectively, the
anatomical pelvic plane P1 and the fixed reference plane P2 are substantially parallel. The fourth pressing surface F 4 is also substantially parallel to the anatomical pelvic plane P1.

The stand 30 has an attachment surface 32A (second surface) for mounting the angle indicator 6. In the examples of FIGS. 4 and 5, the attachment surface 32A is formed at the distal end of the second portion 32 remote from the fixing base 20. Furthermore, the attachment surface 32A extends in a position further away from the fourth pressing surface F4 than the fixed reference plane P2. The attachment surface 32A is a surface parallel to the upper surface 20A and the bottom surface 20B (or the placement surface PB) of the fixing base 20, for example.

In the examples shown in FIG. 4 and FIG. 5, the support mechanisms 51 to 53 respectively support the pressing tools 41 to 43 in such a manner that the pressing surfaces F1 to F3 are movable (slidable) along the third direction D3.

Specifically, the first support mechanism 51 includes a slide groove 51a formed in the second portion 32 of the stand 30 along the third direction D3, a knob 51b including a male screw, and a pin 51c provided in the first pressing tool 41. The male screw of the knob 51b is passed through the slide groove 51a and fastened to a female screw formed in the first pressing tool 41. The pin 51c is also passed through the slide groove 51a.

In the examples of FIGS. 4 and 5, the knob 51b is positioned on the side of the first side face SF1, and the first pressing tool 41 is positioned on the side of the second side face SF2. The first pressing tool 41 is attached to the stand 30 by clamping the second portion 32 together with the knob 51b. The rotation of the first pressing tool 41 is prevented by the pin 51c which is separated from the male screw of the knob 51b.

Similarly, the second support mechanism 52 includes a slide groove 52a formed in the second portion 32 of the stand 30 along the third direction D3, a knob 52b including a male screw, and a pin 52c provided in the second pressing tool 42. The male screw of the knob 52b is passed through the slide groove 52a and fastened to a female screw formed in the second pressing tool 42. The pin 52c is also passed through the slide groove 52a.

In the examples of FIGS. 4 and 5, the knob 52b is positioned on the first side face SF1 side and the second pressing tool 42 is positioned on the second side face SF2 side. The second pressing tool 42 is attached to the stand 30 by clamping the second portion 32 together with the knob 52b. The rotation of the second pressing tool 42 is prevented by the pin 52c which is separated from the male screw of the knob 52b.

As shown in FIG. 4, the third support mechanism 53 includes a first slide groove 53a formed in the second portion 32 of the stand 30 along the third direction D3, a first knob 53b including a male screw, and a first pin 53c. Furthermore, as shown in FIG. 5, the third support mechanism 53 includes an arm 53d that connects the second portion 32 of the stand 30 and the third pressing tool 43, a second slide groove 53e that is formed in the arm 53d along the second direction D2, a second knob 53f including a male screw, and a second pin 53g provided in the third pressing tool 43. The first pin 53c is provided on the arm 53d. The male screw of the first knob 53b is passed through the first slide groove 53a and fastened to a female screw formed in the arm 53d. The first pin 53c is also passed through the first slide groove 53a. The male screw of the second knob 53f is passed through the second slide groove 53e and fastened to a female screw formed in the third pressing tool 43. The second pin 53g is also passed through the second slide groove 53e.

In the examples of FIG. 4 and FIG. 5, the first knob 53b is positioned on the first side face SF1 side, and the third pressing tool 43 and the arm 53d are positioned on the second side face SF2 side. The third pressing tool 43 is attached to the stand 30 by clamping the second portion 32 between the arm 53d and the first knob 53b. The rotation of the arm 53d is prevented by the first pin 53c separated from the male screw of the first knob 53b.

In the examples shown in FIGS. 4 and 5, the arm 53d is in the form of a plate, the second knob 53f is positioned on one side surface of the arm 53d, and the third pressing tool 43 is positioned on the other side surface of the arm 53d. The third pressing tool 43 is attached to the arm 53d by clamping the arm 53d together with the second knob 53f. The rotation of the third pressing tool 43 is prevented by the second pin 53g which is separated from the male screw of the second knob 53f.

FIG. 6 is a view schematically showing the pressing surfaces F1 to F3 and the fixed reference plane P2. When the fastening between the male screw of the knob 51b and the female screw of the first pressing tool 41 is loosened, the first pressing tool 41 can slide in the third direction D3 within a range in which the male screw of the knob 51b and the pin 51c are movable within the slide groove 51a. Similarly, when the fastening between the male screw of the knob 52b and the female screw of the second pressing tool 42 is loosened, the second pressing tool 42 can slide in the third direction D3 within a range in which the male screw of the knob 52b and the pin 52c are movable within the slide groove 52a.

By sliding the first pressing tool 41 and the second pressing tool 42 in this manner, the positions of the first pressing surface F1 and the second pressing surface F2 are shifted along a first reference line SL1 passing through the pressing surfaces F1 and F2. The first reference line SL1 is, for example, parallel to the third direction D3.

When the fastening between the male screw of the first knob 53b and the female screw of the arm 53d is loosened, the third pressing tool 43 can slide in the third direction D3 within a range in which the male screw of the first knob 53b and the first pin 53c are movable within the first slide groove 53a. By sliding the third pressing tool 43 in this manner, the position of the third pressing surface F3 can be adjusted in a direction parallel to the first reference line SL1.

Furthermore, when the fastening between the male screw of the second knob 53f and the female screw of the third pressing tool 43 is loosened, the third pressing tool 43 can slide in the second direction D2 within a range in which the male screw of the second knob 53f and the second pin 53g are movable within the second slide groove 53e. By sliding the third pressing tool 43 in this manner, the position of the third pressing surface F3 can be adjusted along a second reference line SL2 perpendicularly intersecting the first reference line SL1.

In this way, each of the pressing surfaces F1 to F3 can be positioned at a discretionary position on the fixed reference plane P2. Accordingly, it is possible to accurately fix the patient in the lateral position regardless of the physical size etc.

In the configuration of the third support mechanism 53 in this embodiment, the third pressing tool 43 can be detached from the second portion 32 of the stand 30 and attached to the surface on the opposite side of the second portion 32. Accordingly, the fixing device 2 can be used for both a patient lying on the bed B with the right side down, and a patient lying on the bed B with the left side down.

Subsequently, details of the angle indicator 6 will be described.

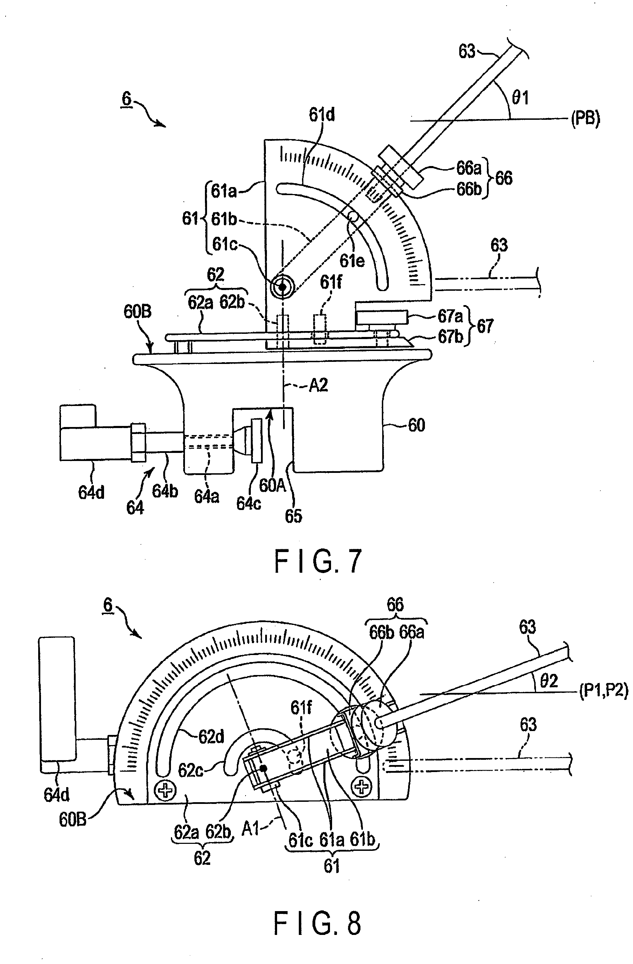

FIG. 7 is a schematic front view of the angle indicator 6, and FIG. 8 is a schematic plan view of the angle indicator 6. As shown in FIG. 7, the indicator base 60 has an attachment groove 65 in its lower portion. The attachment groove 65 includes a flat attachment surface 60A (third surface). The indicator base 60 is fixed by the base fixing mechanism 64 in a state in which the attachment surface 60A is in surface contact with the attachment surface 32A of the stand 30.

In the example shown in FIG. 7, the base fixing mechanism 64 includes a female screw 64a extending through the indicator base 60 from the outer surface to the attachment groove 65, a bolt 64b that is a male screw screwed into the female screw 64a, a pad 64c attached to one end of the bolt 64b located inside the attachment groove 65, and a lever 64d attached to one end of the bolt 64b located outside the attachment groove 65. By rotating the lever 64d in a predetermined direction, the pad 64c moves toward the opposing wall surface of the attachment groove 65, and the second portion 32 of the stand 30 is clamped between the pad 64c and the wall surface. As a result, the angle indicator 6 is fixed to the stand 30. Furthermore, by rotating the lever 64d in the reverse direction, the angle indicator 6 can be removed from the stand 30.

As shown in FIG. 7 and FIG. 8, the first angle adjuster 61 includes a pair of first guide plates 61a, a rod arm 61b, and a first rotation pin 61c. The first guide plate 61a is an example of a plate-shaped first guide member. The first guide plates 61a each have, for example, a fan shape with an internal angle of 90 degrees and fixed to each other. Each of the first guide plates 61a is marked with a scale indicating an angle along an arc, for example. The rod arm 61b is disposed between the first guide plates 61a. The first rotation pin 61c attaches the rod arm 61b to each first guide plate 61a. The rod arm 61b is rotatable about the first rotation pin 61c.

Each first guide plate 61a includes an arc-shaped guide groove 61d. Furthermore, the rod arm 61b has a pair of pins 61e inserted into the respective guide grooves 61d. When the rod arm 61b is rotated, the pins 61e are guided to the guide groove 61d. The indicating rod 63 is attached to the rod arm 61b. The axis of the rod arm 61b and the axis of the indicating rod 63 are on the same straight line.

As shown in FIG. 7 and FIG. 8, the second angle adjuster 62 includes a second guide plate 62a and a second rotation pin 62b. The second guide plate 62a is an example of a plate-shaped second guide member. The second guide plate 62a has, for example, a semicircular arc shape and is fixed to an upper surface 60B of the indicator base 60. The upper surface 60B is parallel to the attachment surface 60A, and the second guide plate 62a is parallel to the upper surface 60B. In the example of FIG. 8, the upper surface 60B has a semicircular arc shape and a scale indicating an angle is provided along an arc. Each first guide plate 61a of the first angle adjuster 61 is rotatably attached to the second guide plate 62a by the second rotation pin 62b.

The second guide plate 62a includes arc-shaped guide grooves 62c and 62d. When the first angle adjuster 61 is rotated, the pin 61f fixed to each first guide plate 61a is guided to the guide groove 62c.

According to the above configuration, the indicating rod 63 is rotatable about a first axis A1 (see FIG. 8) which is the rotation axis of the first rotation pin 61c. As a result, as shown in FIG. 7, it is possible to adjust the first angle θ1 formed by the placement surface PB (or each of the surfaces 20A, 20B, 32A, and 60A described above) and the indicating rod 63. The first axis A1 is parallel to, for example, the second reference line SL2 (or each of the surfaces 20A, 20B, 32A, and 60A described above).

Furthermore, the indicating rod 63 is rotatable about a second axis A2 (see FIG. 7), which is the rotation axis of the second rotation pin 62b. As a result, as shown in FIG. 8, it is possible to adjust the second angle θ2 formed by the fixed reference plane P2 (or the anatomical pelvic plane P1) and the indicating rod 63. The second axis A2 is, for example, parallel to the direction intersecting the first axis A1 (or to the first reference line SL1).

As shown in FIG. 7, the angle indicator 6 further includes a first stopper mechanism 66 that restricts the rotation of the indicating rod 63 by the first angle adjuster 61, and a second stopper mechanism 67 that restricts the rotation of the indicating rod 63 by the second angle adjuster 62 (the rotation of each first guide plate 61a).

In the examples of FIG. 7 and FIG. 8, the first stopper mechanism 66 includes a knob 66a including a female screw, and a stopper 66b. A male screw is formed in the vicinity of the end portion of the indicating rod 63 on the side of the rod arm 61b, and this male screw is screwed into the female screw of the knob 66a. The stopper 66b has an opening, and the indicating rod 63 is passed through the opening. The stopper 66b is disposed between the arc-shaped periphery of each first guide plate 61a and the knob 66a.

By rotating the knob 66a in a direction to press the stopper 66b against each of the first guide plates 61a, it is possible to fix the first angle θ1 by restraining the indicating rod 63. Also, by rotating the knob 66a in the reverse direction to separate the stopper 66b from each of the first guide plates 61a, it is possible to adjust the first angle θ1 by releasing the restraint of the indicating rod 63.

In the example of FIG. 7, the second stopper mechanism 67 includes a knob 67a including a male screw and an extending portion 67b connected to each of the first guide plates 61a. The extending portion 67b is located between the indicator base 60 and the second guide plate 62a and has a female screw. The male screw of the knob 67a passes through the guide groove 62d of the second guide plate 62a and is screwed into the female screw of the extending portion 67b.

By rotating the knob 67a in one direction to tighten the male screw of the knob 67a to the female screw of the extending portion 67b, the second guide plate 62a is clamped between the knob 67a and the extending portion 67b. Accordingly, it is possible to fix the second angle θ2 of the indicating rod 63 by restricting the rotation of the first angle adjuster 61. By rotating the knob 67a in the reverse direction to loosen the fastening of the male screw of the knob 67a and the female screw of the extending portion 67b, the first angle adjuster 61 can be rotated about the second axis A2. Thus, the second angle θ2 of the indicating rod 63 can be adjusted.

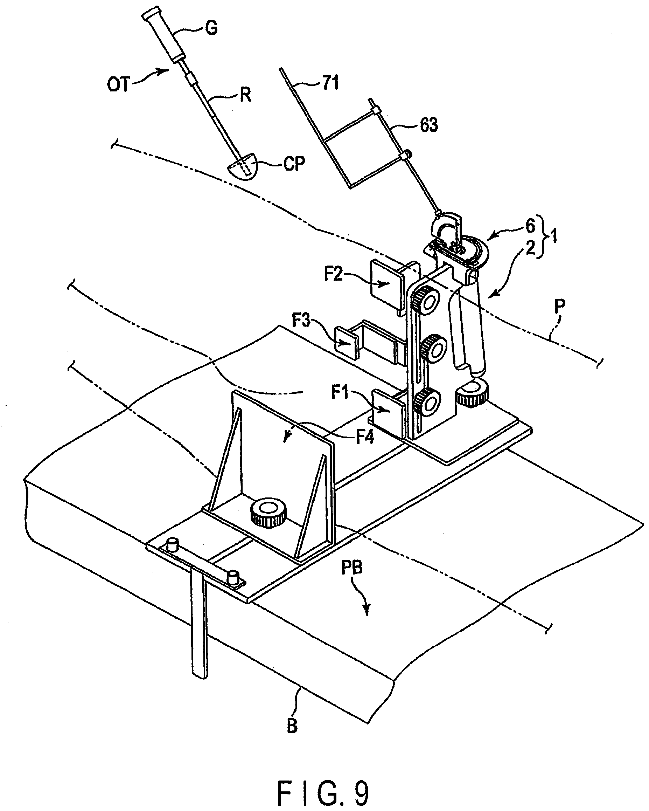

FIG. 9 is a diagram schematically showing an aspect of use of the body position fixing apparatus 1. For treatment of THA, the patient P is placed on the placement surface PB of the bed B in a lateral position. In this state, the patient P is fixed by the fixing device 2. That is, the first pressing surface F1 and the second pressing surface F2 are respectively pressed against the pair of anterior superior iliac spines from the front of the lumbar region, the third pressing surface F3 is pressed against the pubic joint from the front of the lumbar region, and the fourth pressing surface F4 is pressed against the sacrum from the back of the lumbar region.

The cup CP is placed in the acetabulum of the ilium using a surgical instrument OT. The surgical instrument OT includes, for example, a grip G and a rod R extending from the grip G, as shown in the figure. The cup CP is attached to the tip of the rod R so that the normal direction of the cup CP and the axial direction of the rod R coincide with each other. The cup CP is implanted into the acetabulum of the patient P, which has been incised and exposed by the surgical instrument OT.

When placing the cup CP, the angle indicator 6 is used to precisely match the normal cup direction with a preset target angle. That is, when the angles θ1 and θ2 are set as the target angles by the respective angle adjusters 61 and 62, the indicating rod 63 indicates these target angles. The operator can place the cup CP with high precision by implanting the cup CP while keeping the rod R of the surgical instrument OT parallel to the indicating rod 63.

The assisting rod 71 is parallel to the indicating rod 63. Therefore, the operator may adjust the angle of the rod R of the surgical instrument OT with reference to the assisting rod 71. By rotating the assisting rod 71 about the indicating rod 63 so as to approach the rod R, the angle of the rod R can be easily adjusted to the target angle.

The inventor measured the anteversion angles and inclination angles of the placed cup CP based on CT images of the postoperative patient in the case where THA was carried out while fixing the patient in a lateral position with the fixing device 2. Here, the anteversion angle is an index indicating the inclination of the cup normal direction toward the front of the pelvis. The inclination angle is an index indicating the inclination of the cup normal direction toward a side of the pelvis.

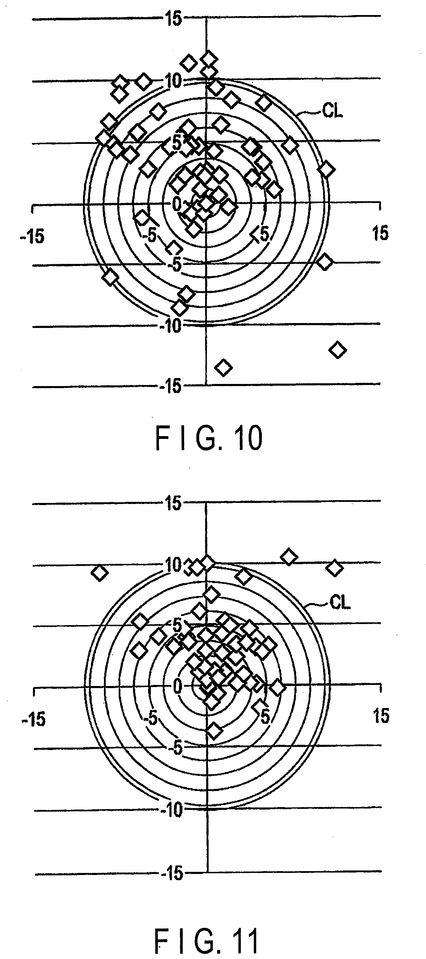

FIG. 10 is a graph showing measurement results obtained in a case where the cup was placed without using the angle indicator 6. In this case, cup placement is performed using, for example, an angle meter (not shown) attached to the surgical instrument OT. FIG. 11 is a graph showing measurement results obtained in a case where the cup was placed using the angle indicator 6. In both graphs, the vertical axis represents the difference (deg) between the target anteversion angle and the measured anteversion angle, and the horizontal axis represents the difference (deg) between the target inclination angle and the measured inclination angle. Rhomboid plots are measurement results, and concentric circles are used for comparison with plot positions. The outermost circle CL passes through points of (anteversion angle, inclination angle) = (± 10, 0), (0, ± 10).

In the graph of FIG. 10, the plots located inside the circle CL are 80% of all plots. In contrast, in the graph of FIG. 11, the plots located inside the circle CL are 94% of all plots. Based on this result, it can be seen that using the angle indicator 6 greatly improves the cup placement accuracy.

The fixing device 2 according to the present embodiment presses the pressing surfaces F1 to F3 against the pair of anterior superior iliac spines and the pubic symphysis, respectively, to fix the patient's posture. In contrast, the conventional fixing device does not include a pressing surface to be pressed against the pubic symphysis. The inventor examined the difference in the cup placement accuracy between the case of using such a conventional fixing device and the case of using the fixing device 2 according to this embodiment. As a result, it was found that when the fixing device 2 according to the present embodiment was used, the cup placement accuracy was improved even when the angle indicator 6 was not used.

In recent years, in order to improve the cup placement accuracy, a navigation system, which detects a position of the patient during surgery and a position of the surgical instrument and navigates the surgical operation, has been introduced. Such a navigation system is effective for improving the cup placement accuracy, but on the other hand, the introduction cost is very high. In addition, work for navigation is necessary, and operation time tends to be long. On the other hand, with the body position fixing apparatus 1 according to the present embodiment, the introduction cost can be kept low and no complicated operation is necessary, so that the operation time can be shortened.

In addition to the above description, various preferred effects can be obtained from this embodiment.

The configuration disclosed by this embodiment can be appropriately modified.

For example, in this embodiment, the body position fixing apparatus 1 that includes the fixing device 2 including the four pressing surfaces F1 to F4 is disclosed. However, the body position fixing apparatus may include a fixing device having more pressing surfaces, fewer pressing surfaces, or a pressing surface to be pressed against a portion other than the anterior superior iliac spines and pubic symphysis.

The structures of the support mechanisms 51 to 53 of the fixing device 2 are not limited to those described with reference to FIG. 4 and FIG. 5. For example, each of the support mechanisms 51 to 53 in the present embodiment has a structure in which each of the surfaces F1 to F3 can slide to a discretionary position; however, each of the support mechanisms 51 to 53 may have a structure that allows each of the surface F1 to F3 to move between a plurality of predetermined positions.

The structure of the rotation mechanism 90 included in the angle indicator 6 is not limited to that described with reference to FIG. 7 and FIG. 8. For example, the rotation mechanism 90 may support the indicating rod 63 so that it can be adjusted to a discretionary angle by a structure similar to a ball joint.

The present embodiment is mainly based on the assumption that the body position fixing apparatus 1 is used for treatment of THA. However, the body position fixing apparatus 1, the fixing device 2, or the angle indicator 6 can also be used for operation, treatment, inspection, etc. other than THA.

## Claims

1. A body position fixing apparatus (1) that fixes a human body placed in a lateral position on a placement surface (PB), the apparatus (1) comprising:
a first pressing tool (41) including a first pressing surface (F1) to be pressed against one of a pair of anterior superior iliac spines (AS) of the human body, that is closer to the placement surface (PB);
a second pressing tool (42) including a second pressing surface (F2) to be pressed against another one of the pair of anterior superior iliac spines (AS), that is more distant from the placement surface (PB);
a third pressing tool (43) including a third pressing surface (F3) to be pressed against a pubic symphysis (PS) of the human body;
a fourth pressing tool (44) including a fourth pressing surface (F4) that faces the first pressing surface (F1), the second pressing surface (F2), and the third pressing surface (F3), and that is to be pressed against a back of a lumbar region of the human body;
a stand (30) to which the first pressing tool (41), the second pressing tool (42), and the third pressing tool (43) are attached; and
an angle indicator (6) including:
an indicating rod (63);
a rotation mechanism (90) that supports the indicating rod (63) to make the indicating rod (63) rotatable at a discretionary angle relative to each of the placement surface (PB) and a fixed reference plane (P2) including the first pressing surface (F1), the second pressing surface (F2), and the third pressing surface (F3); and
a first base (60) attached to the stand (30),
the rotation mechanism (90) including
a first angle adjuster (61) including a first guide member (61a) to which the indicating rod (63) is attached to be rotatable about a first axis (A1) that is parallel to a direction intersecting a reference line (SL1) passing the first pressing surface (F1) and the second pressing surface (F2);
**characterized in that** the rotation mechanism (90) further includes:
a second angle adjuster (62) including a second guide member (62a) which is fixed to the first base (60) and to which the first guide member (61a) is attached so that the first angle adjuster (61) is rotatable about a second axis (A2) that is parallel to the reference line (SL1); and
a first stopper mechanism (66) that restricts the rotation of the indicating rod (63) by the first angle adjuster (61).

2. The body position fixing apparatus (1) of claim 1, further comprising:
a first support mechanism (51) that supports the first pressing tool (41) so that a position of the first pressing surface (F1) is adjustable along the reference line (SL1) passing the first pressing surface (F1) and the second pressing surface (F2);
a second support mechanism (52) that supports the second pressing tool (42) so that a position of the second pressing surface (F2) is adjustable along the reference line (SL1); and
a third support mechanism (53) that supports the third pressing tool (43) so that a position of the third pressing surface (F3) is adjustable along a direction (D3) parallel to the reference line (SL1).

3. The body position fixing apparatus (1) of claim 2, wherein
the third support mechanism (53) supports the third pressing tool (43) so that the position of the third pressing surface (F3) is adjustable along a direction intersecting the reference line (SL1) in addition to the direction (D3) parallel to the reference line (SL1).

4. The body position fixing apparatus (1) of any one of claims 1 to 3, wherein:
the second guide member (62a) is plate-shaped;
the first axis (A1) is parallel to the second guide member (62a); and
the second axis (A2) perpendicularly intersects the second guide member (62a).

5. The body position fixing apparatus (1) of any one of claims 1 to 4, wherein:
the first guide member (61a) includes an arc-shaped periphery;
the indicating rod (63) includes a first male screw;
the first stopper mechanism (66) includes a first knob (66a) including a first female screw into which the first male screw is screwed, and a stopper (66b) disposed between the periphery of the first guide member (61a) and the first knob (66a), and wherein
the indicating rod (63) is restrained by rotating the first knob (66a) in one direction to press the stopper (66b) against the periphery of the first guide member (61a), and restraint of the indicating rod (63) is released by rotating the first knob (66a) in a reverse direction.

6. The body position fixing apparatus (1) of any one of claims 1 to 5, further comprising a second stopper mechanism (67), wherein the second guide member (62a) includes an arc-shaped guide groove (62d),
the second stopper mechanism (67) including:
an extending portion (67b) connected to the first guide member (61a), located between the first base (60) and the second guide member (62a), and including a second female screw; and
a second knob (67a) including a second male screw screwed into the second female screw through the guide groove (62d), and wherein
the first angle adjuster (61) is restrained by clamping the second guide member (62a) with the second knob (67a) and the extending portion (67b) by rotating the second knob (67a) in one direction, and restraint of the first angle adjuster (61) is released by rotating the second knob (67a) in a reverse direction.

7. The body position fixing apparatus (1) of any one of claims 1 to 6, further comprising an assisting rod (71) pivotally supported by the indicating rod (63), wherein the indicating rod (63) and the assisting rod (71) are parallel to each other.

8. The body position fixing apparatus (1) of claim 7, further comprising a shaft (72), one end of which is connected to the assisting rod (71), and another end of which includes an opening that allows passage of the indicating rod (63), wherein the assisting rod (71) and the shaft (72) are movable along the indicating rod (63) and rotatable about the indicating rod (63) as an axis.

9. The body position fixing apparatus (1) of claim 8, further comprising a rod fixing mechanism (73) that is provided in the shaft (72) and fixes the assisting rod (71) to the indicating rod (63), wherein
the assisting rod (71) and the shaft (72) are movable along the indicating rod (63) and rotatable about the indicating rod (63) as the axis, in a state in which fixation by the rod fixing mechanism (73) is released.

10. The body position fixing apparatus (1) of claim 9, further comprising a shaft (72) paired with the shaft (72), the paired shafts (72) being equal in length and being parallel to each other, wherein
the rod fixing mechanism (73) is provided in one of the paired shafts (72) that is closer to the first axis (A1), and is not provided in the other shaft (72).

11. The body position fixing apparatus (1) of claim 6, further comprising a second base (20) including a first surface (20A) on which the fourth pressing tool (44) and the stand (30) are disposed, wherein:
the stand (30) includes a second surface (32A) parallel to the first surface (20A) at a distal portion apart from the second base (20), and in a position further away from the fourth pressing surface (F4) than the fixed reference plane (P2); and
the first base (60) includes a third surface in contact with the second surface (32A),
the apparatus (1) further comprising a fixing mechanism (64) that fixes the first base (60) to the stand (30).

12. The body position fixing apparatus (1) of any one of claims 1 to 11, wherein the first pressing surface (F1), the second pressing surface (F2), and the third pressing surface (F3) are located in one plane (P2),
the apparatus (1) lacking a mechanism that adjusts positions of the first pressing surface (F1), the second pressing surface (F2), and the third pressing surface (F3) in a normal direction (D1) of the fixed reference plane (P2).

13. An angle indicator (6) comprising:
an indicating rod (63);
a base (60) attachable to a fixing device (2) that fixes a human body placed in a lateral position on a placement surface (PB); and
a rotation mechanism (90) that is provided on the base (60) and that supports the indicating rod (63) to make the indicating rod (63) rotatable at a discretionary angle relative to each of the placement surface (PB) and an anatomical pelvic plane (P1) including at least a pair of anterior superior iliac spines (AS) and a pubic symphysis of the human body,
the rotation mechanism (90) including
a first angle adjuster (61) including a first guide member (61a) to which the indicating rod (63) is attached to be rotatable about a first axis (A1);
**characterized in that** the rotation mechanism (90) further includes:
a second angle adjuster (62) including a second guide member (62a) which is fixed to the base (60) and to which the first guide member (61a) is attached so that the first angle adjuster (61) is rotatable about a second axis (A2) that is parallel to a direction intersecting the first axis (A1); and
a first stopper mechanism (66) that restricts the rotation of the indicating rod (63) by the first angle adjuster (61).

14. The angle indicator (6) of claim 13, wherein:
the first guide member (61a) includes an arc-shaped periphery;
the indicating rod (63) includes a first male screw;
the first stopper mechanism (66) includes a first knob (66a) including a first female screw into which the first male screw is screwed, and a stopper (66b) disposed between the periphery of the first guide member (61a) and the first knob (66a), and wherein
the indicating rod (63) is restrained by rotating the first knob (66a) in one direction to press the stopper (66b) against the periphery of the first guide member (61a), and restraint of the indicating rod (63) is released by rotating the first knob (66a) in a reverse direction.

15. The angle indicator (6) of claim 13 or 14, further comprising a second stopper mechanism (67), wherein the second guide member (62a) includes an arc-shaped guide groove (62d),
the second stopper mechanism (67) including:
an extending portion (67b) connected to the first guide member (61a), located between the base (60) and the second guide member (62a), and including a second female screw; and
a second knob (67a) including a second male screw screwed into the second female screw through the guide groove (62d), and wherein the first angle adjuster (61) is restrained by clamping the second guide member (62a) with the second knob (67a) and the extending portion (67b) by rotating the second knob (67a) in one direction, and restraint of the first angle adjuster (61) is released by rotating the second knob (67a) in a reverse direction.

## Patentansprüche

1. Körperpositionsfixiervorrichtung (1), die einen menschlichen Körper fixiert, der in einer Seitenlage auf einer Platzierfläche (PB) platziert ist, die Vorrichtung (1) mit:
einem ersten Drückwerkzeug (41), das eine erste Drückfläche (F1) hat, die gegen einen von einem Paar vorderer oberer Darmbeinstachel (AS) des menschlichen Körpers gedrückt werden soll, der näher an der Platzierfläche (PB) ist;
einem zweiten Drückwerkzeug (42), das eine zweite Drückfläche (F2) hat, die gegen den anderen des Paars vorderer oberer Darmbeinstachel (AS) gedrückt werden soll, der weiter entfernt von der Platzierfläche (PB) ist;
einem dritten Drückwerkzeug (43), das eine dritte Drückfläche (F3) hat, die gegen eine Schambeinfuge (PS) des menschlichen Körpers gedrückt werden soll;
einem vierten Drückwerkzeug (44), das eine vierte Drückfläche (F4) hat, die der ersten Drückfläche (F1), der zweiten Drückfläche (F2) und der dritten Drückfläche (F3) zugewandt ist, und die gegen eine Rückseite einer Lendenwirbelsäule des menschlichen Körpers gedrückt werden soll;
einem Gestell (30), an welchem das erste Drückwerkzeug (41), das zweite Drückwerkzeug (42) und das dritte Drückwerkzeug (43) angebracht sind; und
einem Winkelanzeiger (6), der:
einen Anzeigestab (63);
einen Drehmechanismus (90), der den Anzeigestab (63) stützt, sodass er den Anzeigestab (63) mit einem beliebigen Winkel relativ zu jeder von der Platzierfläche (PB) und einer Fixierbezugsebene (P2) drehbar macht, die die erste Drückfläche (F1), die zweite Drückfläche (F2) und die dritte Drückfläche (F3) enthält; und
eine erste Basis (60) hat, die an dem Gestell (30) angebracht ist,
wobei der Drehmechanismus (90)
eine erste Winkeleinstelleinrichtung (61) hat, die ein erstes Führungsbauteil (61a) hat, an welchem der Anzeigestab (63) angebracht ist, sodass er um eine erste Achse (A1) drehbar ist, die parallel zu einer Richtung ist, die eine Bezugslinie (SL1) schneidet, die die erste Drückfläche (F1) und die zweite Drückfläche (F2) passiert;
**dadurch gekennzeichnet, dass** der Drehmechanismus (90) ferner:
eine zweite Winkeleinstelleinrichtung (62), die ein zweites Führungsbauteil (62a) hat, welches an der ersten Basis (60) fixiert ist und an welchem das erste Führungsbauteil (61a) angebracht ist, sodass die erste Winkeleinstelleinrichtung (61) um eine zweite Achse (A2) drehbar ist, die parallel zu der Bezugslinie (SL1) ist; und
einen ersten Arretiermechanismus (66) hat, der die Drehung des Anzeigestabs (63) mittels der ersten Winkeleinstelleinrichtung (61) begrenzt.

2. Körperpositionsfixiervorrichtung (1) nach Anspruch 1, ferner mit:
einem ersten Stützmechanismus (51), der das erste Drückwerkzeug (41) stützt, sodass eine Position der ersten Drückfläche (F1) entlang der Bezugslinie (SL1) einstellbar ist, die die erste Drückfläche (F1) und die zweite Drückfläche (F2) passiert;
einem zweiten Stützmechanismus (52), der das zweite Drückwerkzeug (42) stützt, sodass eine Position der zweiten Drückfläche (F2) entlang der Bezugslinie (SL1) einstellbar ist; und
einem dritten Stützmechanismus (53), der das dritte Drückwerkzeug (43) stützt, sodass eine Position der dritten Drückfläche (F3) entlang einer Richtung (D3) einstellbar ist, die parallel zu der Bezugslinie (SL1) ist.

3. Körperpositionsfixiervorrichtung (1) nach Anspruch 2, wobei
der dritte Stützmechanismus (53) das dritte Drückwerkzeug (43) stützt, sodass die Position der dritten Drückfläche (F3) zusätzlich zu der Richtung (D3), die parallel zu der Bezugslinie (SL1) ist, entlang einer Richtung einstellbar ist, die die Bezugslinie (SL1) schneidet.

4. Körperpositionsfixiervorrichtung (1) nach einem der Ansprüche 1 bis 3, wobei:
das zweite Führungsbauteil (62a) plattenförmig ist;
die erste Achse (A1) parallel zu dem zweiten Führungsbauteil (62a) ist; und
die zweite Achse (A2) das zweite Führungsbauteil (62a) senkrecht schneidet.

5. Körperpositionsfixiervorrichtung (1) nach einem der Ansprüche 1 bis 4, wobei:
das erste Führungsbauteil (61a) einen bogenförmigen äußeren Rand hat;
der Anzeigestab (63) ein erstes Außengewinde hat;
der erste Arretiermechanismus (66) einen ersten Drehknopf (66a), der ein erstes Innengewinde hat, in welches das erste Außengewinde eingeschraubt ist, und eine Arretiereinrichtung (66b) hat, die zwischen dem äußeren Rand des ersten Führungsbauteils (61a) und dem ersten Drehknopf (66a) angeordnet ist, und wobei
der Anzeigestab (63) durch Drehen des ersten Drehknopfs (66a) in einer Richtung, um die Arretiereinrichtung (66b) gegen den äußeren Rand des ersten Führungsbauteils (61) zu drücken, arretiert wird, und die Arretierung des Anzeigestabs (63) durch Drehen des ersten Drehknopfs (66a) in einer entgegengesetzten Richtung gelöst wird.

6. Körperpositionsfixiervorrichtung (1) nach einem der Ansprüche 1 bis 5, ferner mit einem zweiten Arretiermechanismus (67), wobei das zweite Führungsbauteil (62a) eine bogenförmige Führungsnut (62d) hat,
wobei der zweite Arretiermechanismus (67):
einen Verlängerungsabschnitt (67b), der mit dem ersten Führungsbauteil (61a) verbunden ist, zwischen der ersten Basis (60) und dem zweiten Führungsbauteil (62a) gelegen ist und ein zweites Innengewinde hat; und
einen zweiten Drehknopf (67a) hat, der ein zweites Außengewinde hat, das durch die Führungsnut (62d) in das zweite Innengewinde eingeschraubt ist, und wobei
die erste Winkeleinstelleinrichtung (61) arretiert wird, indem das zweite Führungsbauteil (62a) mit dem zweiten Drehknopf (67a) und dem Verlängerungsabschnitt (67b) durch Drehen des zweiten Drehknopfs (67a) in einer Richtung festgeklemmt wird, und die Arretierung der ersten Winkeleinstelleinrichtung (61) durch Drehen des zweiten Drehknopfs (67a) in einer entgegengesetzten Richtung gelöst wird.

7. Körperpositionsfixiervorrichtung (1) nach einem der Ansprüche 1 bis 6, ferner mit einem Hilfsstab (71), der durch den Anzeigestab (63) schwenkbar gestützt ist, wobei der Anzeigestab (63) und der Hilfsstab (71) parallel zueinander sind.

8. Körperpositionsfixiervorrichtung (1) nach Anspruch 7, ferner mit einer Stange (72), wovon ein Ende mit dem Hilfsstab (71) verbunden ist, und wovon ein anderes Ende eine Öffnung hat, die das Hindurchgehen des Anzeigestabs (63) ermöglicht, wobei der Hilfsstab (71) und die Stange (72) entlang des Anzeigestabs (63) beweglich und um den Anzeigestab (63) als eine Achse drehbar sind.

9. Körperpositionsfixiervorrichtung (1) nach Anspruch 8, ferner mit einem Stabfixiermechanismus (73), der in der Stange (72) vorgesehen ist und den Hilfsstab (71) an dem Anzeigestab (63) fixiert, wobei
der Hilfsstab (71) und die Stange (72) in einem Zustand, in welchem die Fixierung durch den Stabfixiermechanismus (73) gelöst ist, entlang des Anzeigestabs (63) beweglich und um den Anzeigestab (63) als die Achse drehbar sind.

10. Körperpositionsfixiervorrichtung (1) nach Anspruch 9, ferner mit einer Stange (72), die mit der Stange (72) paarweise angeordnet ist, wobei die paarweisen Stangen (72) in ihrer Länge gleich sind und parallel zueinander sind, wobei
der Stabfixiermechanismus (73) an einer der paarweisen Stangen (72) vorgesehen ist, die näher an der ersten Achse (A1) ist, und in der anderen Stange (72) nicht vorgesehen ist.

11. Körperpositionsfixiervorrichtung (1) nach Anspruch 6, ferner mit einer zweiten Basis (20), die eine erste Fläche (20A) hat, an welcher das vierte Drückwerkzeug (44) und das Gestell (30) angeordnet sind, wobei:
das Gestell (30) eine zweite Fläche (32A) hat, die an einem von der zweiten Basis (20) getrennten distalen Abschnitt und in einer Position weiter weg von der vierten Drückfläche (F4) als die Fixierbezugsebene (P2) parallel zu der ersten Fläche (20A) ist; und
die erste Basis (60) eine dritte Fläche hat, die mit der zweiten Fläche (32A) in Berührung steht;
wobei die Vorrichtung (1) ferner einen Fixiermechanismus (64) aufweist, der die erste Basis (60) an dem Gestell (30) fixiert.

12. Körperpositionsfixiervorrichtung (1) nach einem der Ansprüche 1 bis 11, wobei die erste Drückfläche (F1), die zweite Drückfläche (F2) und die dritte Drückfläche (F3) in einer Ebene (P2) liegen,
wobei der Vorrichtung (1) ein Mechanismus fehlt, der Positionen der ersten Drückfläche (F1), der zweiten Drückfläche (F2) und der dritten Drückfläche (F3) in einer normalen Richtung (D1) der Fixierbezugsebene (P2) einstellt.

13. Winkelanzeiger (6) mit:
einem Anzeigestab (63);
einer Basis (60), die an einer Fixiervorrichtung (2) anbringbar ist, die einen menschlichen Körper fixiert, der in einer Seitenlage auf einer Platzierfläche (PB) platziert ist; und
einem Drehmechanismus (90), der auf der Basis (60) vorgesehen ist und der den Anzeigestab (63) stützt, sodass er den Anzeigestab (63) mit einem beliebigen Winkel relativ zu jeder von der Platzierfläche (PB) und einer anatomischen Schambeinebene (P1) drehbar macht, die mindestens ein Paar vorderer oberer Darmbeinstachel (AS) und eine Schambeinfuge des menschlichen Körpers enthält,
wobei der Drehmechanismus (90)
eine erste Winkeleinstelleinrichtung (61) hat, die ein erstes Führungsbauteil (61a) hat, an welchem der Anzeigestab (63) angebracht ist, sodass er um eine erste Achse (A1) drehbar ist;
**dadurch gekennzeichnet, dass** der Drehmechanismus (90) ferner:
eine zweite Winkeleinstelleinrichtung (62), die ein zweites Führungsbauteil (62a) hat, welches an der Basis (60) fixiert ist und an welchem das erste Führungsbauteil (61a) angebracht ist, sodass die erste Winkeleinstelleinrichtung (61) um eine zweite Achse drehbar ist, die parallel zu einer Richtung ist, die die erste Achse (A1) schneidet; und
einen ersten Arretiermechanismus (66) hat, der die Drehung des Anzeigestabs (63) mittels der ersten Winkeleinstelleinrichtung (61) begrenzt.

14. Winkelanzeiger (6) nach Anspruch 13, wobei:
das erste Führungsbauteil (61a) einen bogenförmigen äußeren Rand hat;
der Anzeigestab (63) ein erstes Außengewinde hat;
der erste Arretiermechanismus (66) einen ersten Drehknopf (66a), der ein erstes Innengewinde hat, in welches das erste Außengewinde eingeschraubt ist, und eine Arretiereinrichtung (66b) hat, die zwischen dem äußeren Rand des ersten Führungsbauteils (61a) und dem ersten Drehknopf (66a) angeordnet ist, und wobei
der Anzeigestab (63) durch Drehen des ersten Drehknopfs (66a) in einer Richtung, um die Arretiereinrichtung (66b) gegen den äußeren Rand des ersten Führungsbauteils (61a) zu drücken, arretiert wird, und die Arretierung des Anzeigestabs (63) durch Drehen des ersten Drehknopfs (66a) in einer entgegengesetzten Richtung gelöst wird.

15. Winkelanzeiger (6) nach Anspruch 13 oder 14, ferner mit einem zweiten Arretiermechanismus (67), wobei das zweite Führungsbauteil (62a) eine bogenförmige Führungsnut (62d) hat,
wobei der zweite Arretiermechanismus (67):
einen Verlängerungsabschnitt (67b), der mit dem ersten Führungsbauteil (61a) verbunden ist, zwischen der Basis (60) und dem zweiten Führungsbauteil (62a) gelegen ist und ein zweites Innengewinde hat; und
einen zweiten Drehknopf (67a) hat, der ein zweites Außengewinde hat, das durch die Führungsnut (62d) in das zweite Innengewinde eingeschraubt ist, und wobei die erste Winkeleinstelleinrichtung (61) arretiert wird, indem das zweite Führungsbauteil (62a) mit dem zweiten Drehknopf (67a) und dem Verlängerungsabschnitt (67b) durch Drehen des zweiten Drehknopfs (67a) in einer Richtung festgeklemmt wird, und die Arretierung der ersten Winkeleinstelleinrichtung (61) durch Drehen des zweiten Drehknopfs (67a) in einer entgegengesetzten Richtung gelöst wird.

## Revendications

1. Appareil de fixation de position de corps (1) qui fixe un corps humain placé en position latérale sur une surface de placement (PB), l'appareil (1) comprenant :
un premier outil de pression (41) comportant une première surface de pression (F1) devant être pressée contre l'une d'une paire d'épines iliaques supérieures antérieures (AS) du corps humain, qui est plus proche de la surface de placement (PB) ;
un deuxième outil de pression (42) comportant une deuxième surface de pression (F2) devant être pressée contre une autre de la paire d'épines iliaques supérieures antérieures (AS), qui est plus éloignée de la surface de placement (PB);
un troisième outil de pression (43) comportant une troisième surface de pression (F3) devant être pressée contre une symphyse pubienne (PS) du corps humain ;
un quatrième outil de pression (44) comportant une quatrième surface de pression (F4) qui fait face à la première surface de pression (F1), la deuxième surface de pression (F2) et la troisième surface de pression (F3), et qui doit être pressée contre une partie arrière d'une région lombaire du corps humain ;
un socle (30) auquel le premier outil de pression (41), le deuxième outil de pression (42) et le troisième outil de pression (43) sont attachés ; et
un indicateur d'angle (6) comportant :
une tige indicatrice (63) ;
un mécanisme de rotation (90) qui supporte la tige indicatrice (63) pour rendre la tige indicatrice (63) capable de tourner à un angle discrétionnaire par rapport à chacun(e) de la surface de placement (PB) et d'un plan de référence fixe (P2) comportant la première surface de pression (F1), la deuxième surface de pression (F2) et la troisième surface de pression (F3) ; et
une première base (60) attachée au socle (30),
le mécanisme de rotation (90) comportant
un premier dispositif de réglage d'angle (61) comportant un premier élément de guidage (61a) auquel la tige indicatrice (63) est attachée pour pouvoir tourner autour d'un premier axe (A1) qui est parallèle à une direction coupant une ligne de référence (SL1) passant par la première surface de pression (F1) et la deuxième surface de pression (F2) ;
**caractérisé en ce que** le mécanisme de rotation (90) comporte en outre :
un deuxième dispositif de réglage d'angle (62) comportant un deuxième élément de guidage (62a) qui est fixé à la première base (60) et auquel le premier élément de guidage (61a) est attaché de sorte que le premier dispositif de réglage d'angle (61) puisse tourner autour d'un deuxième axe (A2) qui est parallèle à la ligne de référence (SL1) ; et
un premier mécanisme de butée (66) qui limite la rotation de la tige indicatrice (63) par le premier dispositif de réglage d'angle (61).

2. Appareil de fixation de position de corps (1) de la revendication 1, comprenant en outre :
un premier mécanisme de support (51) qui supporte le premier outil de pression (41) de sorte qu'une position de la première surface de pression (F1) soit réglable le long de la ligne de référence (SL1) passant par la première surface de pression (F1) et la deuxième surface de pression (F2) ;
un deuxième mécanisme de support (52) qui supporte le deuxième outil de pression (42) de sorte qu'une position de la deuxième surface de pression (F2) soit réglable le long de la ligne de référence (SL1) ; et
un troisième mécanisme de support (53) qui supporte le troisième outil de pression (43) de sorte qu'une position de la troisième surface de pression (F3) soit réglable le long d'une direction (D3) parallèle à la ligne de référence (SL1).

3. Appareil de fixation de position de corps (1) de la revendication 2, dans lequel
le troisième mécanisme de support (53) supporte le troisième outil de pression (43) de sorte que la position de la troisième surface de pression (F3) soit réglable le long d'une direction coupant la ligne de référence (SL1) en plus de la direction (D3) parallèle à la ligne de référence (SL1).

4. Appareil de fixation de position de corps (1) de l'une quelconque des revendications 1 à 3, dans lequel :
le deuxième élément de guidage (62a) est en forme de plaque ;
le premier axe (A1) est parallèle au deuxième élément de guidage (62a) ; et
le deuxième axe (A2) coupe perpendiculairement le deuxième élément de guidage (62a).

5. Appareil de fixation de position de corps (1) de l'une quelconque des revendications 1 à 4, dans lequel :
le premier élément de guidage (61a) comporte une périphérie en forme d'arc ;
la tige indicatrice (63) comporte une première vis mâle ;
le premier mécanisme de butée (66) comporte un premier bouton (66a) comportant une première vis femelle dans laquelle la première vis mâle est vissée, et une butée (66b) disposée entre la périphérie du premier élément de guidage (61a) et le premier bouton (66a), et où
la tige indicatrice (63) est retenue en tournant le premier bouton (66a) dans un sens pour presser la butée (66b) contre la périphérie du premier élément de guidage (61a), et la retenue de la tige indicatrice (63) est libérée en tournant le premier bouton (66a) dans le sens inverse.

6. Appareil de fixation de position de corps (1) de l'une quelconque des revendications 1 à 5, comprenant en outre un deuxième mécanisme de butée (67), où le deuxième élément de guidage (62a) comporte une rainure de guidage en forme d'arc (62d),
le deuxième mécanisme de butée (67) comportant :
une partie d'extension (67b) reliée au premier élément de guidage (61a), située entre la première base (60) et le deuxième élément de guidage (62a), et comportant une deuxième vis femelle ; et
un deuxième bouton (67a) comportant une deuxième vis mâle vissée dans la deuxième vis femelle à travers la rainure de guidage (62d), et où
le premier dispositif de réglage d'angle (61) est retenu en serrant le deuxième élément de guidage (62a) avec le deuxième bouton (67a) et la partie d'extension (67b) en tournant le deuxième bouton (67a) dans un sens, et la retenue du premier dispositif de réglage d'angle (61) est libérée en tournant le deuxième bouton (67a) dans le sens inverse.

7. Appareil de fixation de position de corps (1) de l'une quelconque des revendications 1 à 6, comprenant en outre une tige d'assistance (71) supportée en pivotement par la tige indicatrice (63), où la tige indicatrice (63) et la tige d'assistance (71) sont parallèles l'une à l'autre.

8. Appareil de fixation de position de corps (1) de la revendication 7, comprenant en outre un arbre (72), dont une extrémité est reliée à la tige d'assistance (71), et dont une autre extrémité comporte une ouverture qui permet le passage de la tige indicatrice (63), où la tige d'assistance (71) et l'arbre (72) sont mobiles le long de la tige indicatrice (63) et peuvent tourner autour de la tige indicatrice (63) en tant qu'axe.

9. Appareil de fixation de position de corps (1) de la revendication 8, comprenant en outre un mécanisme de fixation de tige (73) qui est prévu dans l'arbre (72) et fixe la tige d'assistance (71) à la tige indicatrice (63), où
la tige d'assistance (71) et l'arbre (72) sont mobiles le long de la tige indicatrice (63) et peuvent tourner autour de la tige indicatrice (63) comme étant l'axe, dans un état dans lequel la fixation par le mécanisme de fixation de tige (73) est libérée.

10. Appareil de fixation de position de corps (1) de la revendication 9, comprenant en outre un arbre (72) associé à l'arbre (72), les arbres associés (72) étant de longueur égale et étant parallèles entre l'un à l'autre, où
le mécanisme de fixation de tige (73) est prévu dans l'un des arbres associés (72) qui est plus proche du premier axe (A1), et n'est pas prévu dans l'autre arbre (72).

11. Appareil de fixation de position de corps (1) de la revendication 6, comprenant en outre une deuxième base (20) comportant une première surface (20A) sur laquelle le quatrième outil de pression (44) et le socle (30) sont disposés, où :
le socle (30) comporte une deuxième surface (32A) parallèle à la première surface (20A) au niveau d'une partie distale séparée de la deuxième base (20), et dans une position plus éloignée de la quatrième surface de pression (F4) que le plan de référence fixe (P2) ; et
la première base (60) comporte une troisième surface en contact avec la deuxième surface (32A),
l'appareil (1) comprenant en outre un mécanisme de fixation (64) qui fixe la première base (60) au socle (30).

12. Appareil de fixation de position de corps (1) de l'une quelconque des revendications 1 à 11, dans lequel la première surface de pression (F1), la deuxième surface de pression (F2) et la troisième surface de pression (F3) sont situées dans un plan (P2),
l'appareil (1) étant dépourvu d'un mécanisme qui règle les positions de la première surface de pression (F1), de la deuxième surface de pression (F2) et de la troisième surface de pression (F3) dans un sens normal (D1) du plan de référence fixe (P2).

13. Indicateur d'angle (6) comprenant :
une tige indicatrice (63) ;
une base (60) pouvant être attachée à un dispositif de fixation (2) qui fixe un corps humain placé en position latérale sur une surface de placement (PB) ; et
un mécanisme de rotation (90) qui est prévu sur la base (60) et qui supporte la tige indicatrice (63) pour rendre la tige indicatrice (63) capable de tourner à un angle discrétionnaire par rapport à chacun(e) de la surface de placement (PB) et d'un plan pelvien anatomique (P1) comprenant au moins une paire d'épines iliaques supérieures antérieures (AS) et une symphyse pubienne du corps humain,
le mécanisme de rotation (90) comportant
un premier dispositif de réglage d'angle (61) comportant un premier élément de guidage (61a) auquel la tige indicatrice (63) est attachée pour pouvoir tourner autour d'un premier axe (A1) ;
**caractérisé en ce que** le mécanisme de rotation (90) comporte en outre :
un deuxième dispositif de réglage d'angle (62) comportant un deuxième élément de guidage (62a) qui est fixé à la base (60) et auquel le premier élément de guidage (61a) est attaché de sorte que le premier dispositif de réglage d'angle (61) puisse tourner autour d'un deuxième axe (A2) qui est parallèle à une direction coupant le premier axe (A1) ; et
un premier mécanisme de butée (66) qui limite la rotation de la tige indicatrice (63) par le premier dispositif de réglage d'angle (61).

14. Indicateur d'angle (6) de la revendication 13, dans lequel :
le premier élément de guidage (61a) comporte une périphérie en forme d'arc ;
la tige indicatrice (63) comporte une première vis mâle ;
le premier mécanisme de butée (66) comporte un premier bouton (66a) comportant une première vis femelle dans laquelle la première vis mâle est vissée, et une butté (66b) disposée entre la périphérie du premier élément de guidage (61a) et le premier bouton (66a), et où
la tige indicatrice (63) est retenue en tournant le premier bouton (66a) dans un sens pour presser la butée (66b) contre la périphérie du premier élément de guidage (61a), et la retenue de la tige indicatrice (63) est libérée en tournant le premier bouton (66a) dans le sens inverse.

15. Indicateur d'angle (6) de la revendication 13 ou 14, comprenant en outre un deuxième mécanisme de butée (67), où le deuxième élément de guidage (62a) comporte une rainure de guidage en forme d'arc (62d),
le deuxième mécanisme de butée (67) comportant :
une partie d'extension (67b) reliée au premier élément de guidage (61a), située entre la base (60) et le deuxième élément de guidage (62a), et comprenant une deuxième vis femelle ; et
un deuxième bouton (67a) comportant une deuxième vis mâle vissée dans la deuxième vis femelle à travers la rainure de guidage (62d), et où le premier dispositif de réglage d'angle (61) est retenu en serrant le deuxième élément de guidage (62a) avec le deuxième bouton (67a) et la partie d'extension (67b) en tournant le deuxième bouton (67a) dans un sens, et la retenue du premier dispositif de réglage d'angle (61) est libérée en tournant le deuxième bouton (67a) dans le sens inverse.
